(19) **Europäisches Patentamt**
**European Patent Office**
**Office européen des brevets**

(11) **EP 1 689 880 B1**

(12) **EUROPEAN PATENT SPECIFICATION**

(45) Date of publication and mention
of the grant of the patent:
**22.04.2009 Bulletin 2009/17**

(51) Int Cl.:
***C12Q 1/26*** *(2006.01)*     ***C12Q 1/42*** *(2006.01)*

(21) Application number: **04819231.4**

(86) International application number:
**PCT/EP2004/013707**

(22) Date of filing: **25.11.2004**

(87) International publication number:
**WO 2005/052185 (09.06.2005 Gazette 2005/23)**

(54) **DETECTION OF NO BY USING GUANYLYL CYCLASE AND CGMP PRODUCTION AS A READOUT SYSTEM**

NACHWEIS VON NO UNTER VERWENDUNG VON GUANYLYLCYCLASE UND CGMP-PRODUKTION ALS READOUT-SYSTEM

DETECTION DE NO PAR UTILISATION DE LA PRODUCTION DE GUANYLYLE CYCLASE ET DE CGMP COMME SYSTEME DE LECTURE

(84) Designated Contracting States:
**AT BE BG CH CY CZ DE DK EE ES FI FR GB GR HU IE IS IT LI LU MC NL PL PT RO SE SI SK TR**

(30) Priority: **26.11.2003 EP 03027159**

(43) Date of publication of application:
**16.08.2006 Bulletin 2006/33**

(73) Proprietors:
• **Boehringer Ingelheim International GmbH**
**55216 Ingelheim am Rhein (DE)**
Designated Contracting States:
**AT BE BG CH CY CZ DK EE ES FI FR GB GR HU IE IS IT LI LU MC NL PL PT RO SE SI SK TR**
• **Boehringer Ingelheim Pharma GmbH & Co.KG**
**55216 Ingelheim am Rhein (DE)**
Designated Contracting States:
**DE**

(72) Inventor: **BÜTTNER, Frank**
**88448 Attenweiler (DE)**

(74) Representative: **Hammann, Heinz et al**
**c/o Boehringer Ingelheim GmbH,**
**CD Patents**
**55216 Ingelheim am Rhein (DE)**

(56) References cited:
**WO-A-02/20831**

• **MAYER BERND ET AL: "A new pathway of nitric oxide/cyclic GMP signaling involving S-nitrosoglutathione" JOURNAL OF BIOLOGICAL CHEMISTRY, vol. 273, no. 6, 6 February 1998 (1998-02-06), pages 3264-3270, XP002320131 ISSN: 0021-9258**

**Description**

Introduction

The L-arginine-nitric oxide pathway is involved in a large variety of physiological processes (Mayer; B. et al., 1997; Vallance, P. et al., 2002; Wendy, K. et al., 2001), like for example the regulation of blood pressure, platelet aggregation or host defence against pathogens. Nitric oxide (NO) can be for example produced by nitric oxide synthase (NOS) representing a group of enzymes existing as three different isoforms of NOS: eNOS, nNOS and iNOS (Janssens, S.P. et al., 1992; Marsden, P.A. et al., 1992; Nakane, M. et al., 1993; Geller, D.A. et al., 1993; Sherman, P.A. et al. 1993; Charles, I.G. et al., 1993; Maier, R. et al., 1994; Chartrain, N.A. et al., 1994).

To influence diseases which are associated with NOS activity (e.g. chronic obstructive pulmonary disease (COPD), arteriosclerosis, wound-heeling) modulation of NOS activity is wanted. Therefore, there exists a need for substances which can modulate NOS activity. Subsequently, there is a need for e.g. an ultra high throughput compatible assay which is sensitive enough to determine whether a substance can modulated NOS activity.

In the following the known reaction of NOS with Arginine in presence of NADPH, FAD, FMN and biopterin resulting in Citrulline and No and NADP$^+$ is given (Griffith, O.W. et al, 1995; Mayer, B. et al.; 1995; Stuehr, D.J. et al., 1991; Klatt, P. et al., 1993; Feldmann, P.L. et al, 1993; Marletta, M.A., et al., 1994; Pufahl, R.A. et al., 1993; Clement, B. et al., 1993;):

$$\textbf{Arginine} + \textbf{NOS} \xrightarrow[\text{biopterin}]{\substack{\textbf{NADPH,}\\\textbf{FAD, FMN,}}} \textbf{Citrulline} + \textbf{NO} + \textbf{NADP}^+$$

To verify whether NOS activity indeed has been altered it is essential to measure NOS activity. To measure the activity of a NOS enzyme it is necessary to detect arginine consumption, citrulline production or NO generation.

Currently, only assay systems which are not compatible to miniaturized high through put screening systems are know. Only radioactive systems for measuring arginine or citrulline are available. (Mayer et al., J. Biol. chem. 1998, vol. 273, No. 6, pp. 326A - 327O). To make matters worse, said systems are only meaningful at high doses of arginine or citrulline and therewith respective miniaturising is not successfully possible.

Other systems are not usable for a screening campaign since a substance to be tested interacts unspecifically with substances of the system (Powell D.and Williams D., 2002).

Since NO is only stable for a few seconds it is not regarded as a suitable read-out. Commercially available NO detection systems have been failed as a suitable readout technique in high throughput screening (Misko T.P. et al. 1993).

For a high throughput compatible NOS activity assay for a drug screening campaign it is necessary to have a sensitive and miniaturizable assay format.

Description Of The Invention

Until now nitric oxide (NO) was regarded as a not suitable readout since it is only stable for a few seconds. The present invention provides for a method for detecting NO which is surprisingly accessible to a new detection method of the present invention.

The guanylyl cyclase is a physiological target for NO. Guanylyl cyclase becomes activated by NO several hundred fold. In the presence of GTP a said activated guanylyl cyclase catalyses the generation of cGMP. Therewith, NO causes the generation of cGMP. As disclosed in the present invention said signal transduction can be surprisingly used in an inverse direction to detect NO quantitatively.

We have established a high throughput compatible assay format for the detection of Nitric oxide synthase (NOS) activity employing guanylyl cyclase and cGMP as an amplification and readout system for the detection of NO. Our assay setup is as follows: recombinantly expressed NOS enzyme, purified NOS enzyme from a natural source or working with a cell based assay system containing NOS is used to generate NO. Adding soluble guanylyl cyclase to the system leads to an increased activation of the guanylyl cyclase enzyme. This results in the generation of cyclic GMP which is then detected using a cGMP detection system. As a cGMP detection system e.g. commercially available detection systems can be used.

This detection system for NO is quite sensitive since the activation of the guanylyl cyclase results in an cGMP increase. One activated guanylyl cyclase can generate several cGMP molecules therefore we have an amplification effect and can overcome the sensitivity issue.

The present invention provides therewith a technical basis for a long felt need: An assay for determining whether a substance can modulate NOS activity. Therewith the invention also provides for a high throughput screening (HTS) compatible NOS activity assay for a drug screening campaign.

**Nitric oxide synthase (NOS)** according to the present invention is any enzyme which is selected from a group consisting of: i-NOS (inducible NO synthase, II) e-NOS (endothelial NO synthase, III), and n-NOS (neuronal NO synthase, I)

**Nitric oxide synthase (NOS) activity** according to the present invention is an activity which catalyzes the formation of NO by converting arginine to citrulline with the concomitant release of NO. NOS activity according to the present invention includes any further conversion of any other substrate resulting in the generation of NO.

**[0015]** **A substrate according to the invention** is every substance which can be transformed by NOS in any other substance as long as NO is produced. A preferred substance of the invention is arginine.

**A substance to be tested** to be a modulator of NOS according to the invention is any small chemical molecule, peptide, or antibody.

**A modulator** according to the invention is either an inhibitor of the invention or an activator of the invention.

**An inhibitor of** the nitric oxide synthase activity according to the present invention is identifiable by an reduced level of cGMP in a method according to the invention which can be measured in the solution to which the substance to be tested (in step (c)) was added.

**An activator** of the nitric oxide synthase activity according to the present invention is identifiable by an enhanced level of cGMP in a method according to the invention which can be measured in the solution to which the substance to be tested (in step (c)) was added.

A method according to the invention is, characterized in that the method comprises:

- providing a solution comprising NO;
- adding guanylyl cyclase and guanosin triphosphate (GTP) under conditions which allow synthesis of cyclic guanosin monophosphate (cGMP);
- measuring cGMP with

an antibody which is capable of binding to cGMP.
Said method can be used to detect NO in solution.
Said method can additionally be used in a high throughput screening. Therefore, the present invention provides for a method for detection of nitric oxide (NO)) in an high throughput (HTS) format characterized in that the above mentioned method is performed. Said method can be performed by using a plate having at least 96 wells, more preferred is the use of a 384 well plate or a 1536 well plate. Also preferred is the use of a chip as reaction and/or readout platform.

In one embodiment the present invention provides for a method for measuring nitric oxide synthase activity, characterized in that the method comprises:

(a) providing a solution comprising a nitric oxide synthase and a suitable substrate under conditions which allow synthesis of nitric oxide;
(b) adding guanylyl cyclase and GTP under conditions which allow synthesis of cGMP;
(c) measuring CGMP with

an antibody which is capable of binding to cGMP.
Said method can be used in a high throughput screening. Therefore, the present invention provides for a method for measuring nitric oxide synthase (NOS) activity in an high throughput (HTS) format characterized in that the above mentioned method is performed. Said method can be performed by using a plate having at least 96 wells, more preferred is the use of a 384 well plate or a 1536 well plate. Also preferred is the use of a chip as reaction and/or readout platform.

In a further embodiment the present invention provides for a method for determining whether a substance is modulator of nitric oxide synthase activity according to the invention, characterized in that the method comprises:

(a) providing a first solution comprising a nitric oxide synthase and a suitable substrate under conditions which allow synthesis of nitric oxide;
(b) providing a second solution comprising a nitric oxide synthase and a suitable substrate under conditions which allow synthesis of nitric oxide;
(c) adding a substance to be tested to the first or the second solution;
(d) adding guanylyl cyclase and GTP under conditions which allow synthesis of cGMP to the first and the second solution;

(e) measuring cGMP in the first and the second solution;

(f) comparing cGMP levels of the first and the second solution, wherein a modulator of a nitric oxide synthase activity is identified by different levels of cGMP.

The method for measuring cGMP according to the invention uses an antibody which is capable of binding to cGMP.

Said method can be used in a high throughput screening. Therefore the present invention provides for a method for determining whether a substance is modulator of nitric oxide synthase (NOS) activity in an high throughput (HTS) format characterized in that the above mentioned method is performed. Said method can be performed by using a plate having at least 96 wells, more preferred is the use of a 384 well plate or a 1536 well plate. Also preferred is the use of a chip as reaction and/or readout platform.

In a further embodiment all above mentioned methods of to the invention additionally comprise superoxid dismutase (SOD) which can be added together with guanylyl cyclase and GTP.

All methods of the present invention can be used in a high throughput screening assays.

The present invention also provides for a kit for determining whether a substance is a modulator - i.e. an activator or an inhibitor - of NOS activity comprising:

guanylyl cyclase, GTP and an antibody which is capable of binding to cGMP.

In a more preferred embodiment said kit of the invention further comprises Superoxide dismutase (SOD).

In a most preferred embodiment said kit of the invention further comprises nitric oxid synthase (NOS) which is selected from a group consisting of: i-NOS, e-NOS, and n-NOS. This kit can additionally comprise Superoxide dismutase (SOD).

In a further embodiment the present invention also provides for a kit for measuring nitric oxide synthase (NOS) activity comprising: guanylyl cyclase, GTP and an antibody which is capable of binding to cGMP.

In a more preferred embodiment said kit of the invention further comprises Superoxide dismutase (SOD).

In a most preferred embodiment said kit of the invention further comprises nitric oxid synthase (NOS) which is selected from a group consisting of: i-NOS, e-NOS, and n-NOS. This kit can additionally comprise Superoxide dismutase (SOD).

The present invention additionally provides for a further kit for detection of nitric oxid (NO) comprising: guanylyl cyclase, GTP and an antibody which is capable of binding to cGMP. In a more preferred embodiment said kit of the invention further comprises Superoxide dismutase (SOD).

The following Examples are meant to illustrate the present invention, however, shall not be construed as limitation. However, the Examples describe most preferred embodiments of the invention.

Examples:

Example 1: Assay description

Detection of NOS activity by using the activation of soluble guanylyl cyclase, an enzyme which is well known for years (Gerzer R., et al. 1981; Zabel U.,et al., 1998; Hoenicka, M.et al., 1999; Lee, Y.C. et al., 2000; Kosarikov, D.N. et al.; 2001). NO produced by the different NOS enzymes can activate soluble guanylyl cyclase and also its membrane standing counterpart. In said type of assays recombinantly expressed NOS, a cell-lysate containing NOS as well as NOS purified from a natural source can be used (Janssens, S.P. et al., 1992; Marsden, P.A. et al., 1992; Nakane, M. et al., 1993; Geller, D.A. et al., 1993; Sherman, P.A. et al. 1993; Charles, I.G. et al., 1993; Maier, R. et al., 1994; Chartrain, N.A. et al., 1994).

Here we describe a non radioactive HTS compatible assay format which can be used to detect the NO produced by the NOS activity via activation of guanylyl cyclase and subsequent detection of the produced cGMP using the AlphaScreen technology in a mix and measure mode.

Arginine as a substrate is converted by each of the three different NOS enzymes respectively, into citrulline. During this process NO is also produced. Therefore NO detection can be used to monitor directly the activity NOS enzymes. It is known that NO can directly stimulate the soluble guanylyl cyclase. This activation leads to an increase in cGMP production by the activated guanylyl cyclase. The produced cGMP can be detected and quantified. A variety of different technologies can be employed for the detection and quantification of the newly produced cGMP. This technologies can be e.g. based on HTRF (Claret E.J., et al. , 2004) or radioactivity (Perkin Elmer: Flashplate cyclic cGMP [125I]-assay product no: SMP002J001PK) or like it is shown here based on the AlphaScreen technology. Using the Alphascreen cGMP competition assay a biotinylated cGMP is used to generate a fluorescence signal. The biotinylated cGMP is binding to the AlphaScreen donor bead via streptavidin and is binding to the AlphaScreen acceptor bead via an anti-cGMP specific antibody bound to the bead surface. If cGMP is freshly produced by the stimulated guanylyl cyclase it is competing with the binding to the anti-cGMP antibody. Therefore, the standard fluorescence signal is getting reduced by freshly produced cGMP, which is not biotinylated.

Example 2: Standard operating procedure for performing a mix and measure HTS compatible NOS activity assay.

**Method**

In 384-well plates, 3 $\mu$l of a test compound diluted in distilled water (final concentration of compounds 5 $\mu$g/ml; DMSO 1%) is mixed with 3 $\mu$l enzyme mix dissolved in assay buffer (i-NOS final 2-10 $\mu$g/ml or e-NOS final 2-10 $\mu$g/ml or n-NOS final 2-10 $\mu$g/ml, sGC final 1:800 000 - given amounts of respective NOS enzymes are based on total concentration of cell culture supernatant employed (insect cell recombinant expression system)). After an incubation time of 30 minutes 3$\mu$l substrate mix dissolved in assay buffer (GTP final 100$\mu$M, Arginine final 3$\mu$M, NADPH final 500$\mu$M, for n-NOS 1 $\mu$M Ca$^{2+}$ and 1 $\mu$M Calmodulin is necessary) is added. After another incubation time of 90 minutes the reaction is stopped with 10 $\mu$l acceptor and donor bead-antibody mix dissolved in stop/detection buffer (final concentration of each bead-type 15 $\mu$g/ml; antibody final 1:15000).
After an overnight incubation, the assay is measured using e.g. an Alphaquest reader (Perkin Elmer), which is measuring fluorescence at 520-620 nm.

| | |
|---|---|
| Assay buffer: | 25mM Tris pH 7.4, 3mM MgCl$_2$, 3mM DTT, 0.05% BSA, 3$\mu$M BH$_4$, 1 $\mu$M FAD, 1$\mu$M FMN, 10$\mu$M 4-{[3', 4'-(Methylenedioxy) benzyl]amino}-6- methoxyquinazoline |
| Stop / detection buffer: | 50mM Tris, 50mM EDTA pH 7.4, 0.10% BSA, 0.10% Tween-20 |

Each assay microtiter plate contains wells with vehicle control instead of compound (1% DMSO in water) as reference for non-inhibited enzyme activity (100% CTL; high values) and wells with 100$\mu$M AMT as reference for inhibited enzyme activity (0% CTL; low values).
Each assay micro-titer plate contains a cGMP standard curve (1, 10, 100, 1000, 3000, 10000, 100000, 1000000 pM/l) in duplicate used as reference to calculate the amount of cGMP produced in every well.
The analysis of the data is performed by the calculation of the percentage of cGMP in the presence of the test compound compared to cGMP generated from the positive control:

$$(cGMP(sample) - cGMP (low value)) \times 100/ (cGMP (high value) - cGMP (low value))$$

An inhibitor of the enzyme activity will give values between 100% Ctl (no inhibition) and 0% Ctl (complete inhibition).
Materials used are well known in the art, however, in the following respective suppliers are given: The 384 low volume plates were purchased from Greiner (white; cat.-no. 784075).The AlphaScreen IgG Detection Kit (cat.-no. 6760617) and the biotinylated cGMP were bought from Perkin Elmer. The anti-cGMP antibody (cat.-no. 970161) is from Merck Biosciences. The different NOS enzymes i-NOS (cat.-no. 201-069), e-NOS (cat.-no. 201-070) and n-NOS (cat.-no. 201-068), sGC (cat.-no. 201-177) and the L-Arginine (cat.-no. 101-004) were from Alexis. The NADPH (cat.-no. N-6005) and the GTP (cat.-no. G-5884) were purchased from Sigma. All other materials were of highest grade commercially available.

Example 3: Standard operating procedure for performing a mix and measure HTS compatible NOS activity assay optimized by adding a further enzyme to the reaction mixture the Superoxide dismutase (SOD)

In the following a further variant of the assay of the invention is disclosed in which an enzyme has been added to the reaction mixture. The Superoxide dismutase (SOD) (Keele Jr. B. B. et al.,1970; Beckman J. S. et al., 1990) increases the sensitivity of the assay system and therefore reduces the amount of NOS enzyme needed for performing the assay.

**Method**

In the 384-well plates; 3 $\mu$l of the test compounds diluted in distilled water (final concentration of compounds 5 $\mu$g/ml; DMSO 1 %) is mixed with 3 $\mu$l enzyme mix dissolved in assay buffer (i-NOS final 2$\mu$g/ml, e-NOS 5 $\mu$g/ml, n-NOs 0.4 $\mu$g/ml, SOD final 300nM, sGC final 1:800 000 - given amounts of respective NOS enzymes are based on total concentration of cell culture supernatant employed (insect cell recombinant expressions system)). After an incubation time of 30 minutes 3$\mu$l substrate mix dissolved in assay buffer ( GTP final 100$\mu$M, Arginine final 3$\mu$M, NADPH final 500$\mu$M, for nNOS 1 $\mu$M Ca$^{2+}$ and 1 $\mu$M Calmodulin is necessary) is added. After another incubation time of 90 minutes the reaction is stopped with 10 $\mu$l acceptor and donor bead-antibody mix dissolved in stop/detection buffer (final concentration of each bead-type 15 $\mu$g/ml; antibody final 1:15000).

After an overnight incubation, the assay is measured using e.g. an Alphaquest (as outlined in Example 2).

| | |
|---|---|
| Assay buffer: | 25mM Tris pH 7.4, 3mM MgCl$_2$, 3mM DTT, 0.05% BSA, 3$\mu$M BH$_4$, 1$\mu$M FAD, 1 $\mu$M FMN, 10$\mu$M 4-{[3', 4'-(Methylenedioxy) benzyl]amino}-6- methoxyquinazoline |
| Stop/ detection buffer: | 50mM Tris, 50mM EDTA pH 7.4, 0.10% BSA, 0.10% Tween-20 |

Each assay microtiter plate contains wells with vehicle control instead of compound (1% DMSO in water) as reference for non-inhibited enzyme activity (100% CTL; high values) and wells with 100$\mu$M AMT as reference for inhibited enzyme activity (0% CTL; low values).

Each assay microtiter plate contains a cGMP standard curve (1, 10, 100, 1000, 3000, 10000, 100000, 1000000 pM/l) in duplicate used as reference to calculate the amount of cGMP produced in every well.

The analysis of the data is performed by the calculation of the percentage of cGMP in the presence of the test compound compared to cGMP generated from the positive control.

$$(cGMP(sample) - cGMP\ (low\ value)) \times 100/\ (cGMP\ (high\ value) - cGMP\ (low\ value))$$

An inhibitor of the enzyme activity will give values between 100% Ctl (no inhibition) and 0% Ctl (complete inhibition).

Example 4: Stimulation of soluble guanylyl cyclase and subsequent cGMP detection:

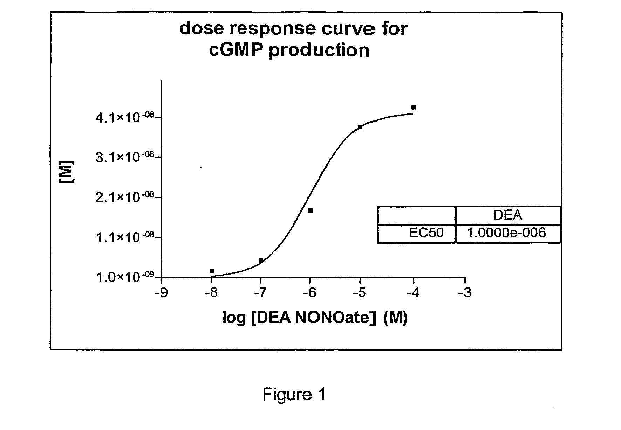

The principle mechanism of the stimulation of the soluble guanylyl cyclase can be shown by using a NO donor compound like for example DEA NONOate or others like DETA NONOate, DPTA NONOate, GEA 5024 or GEA 3162 (Drago R.S. & Paulik F.E., 1960; Drago R.S. & B.R. Karstetter B.R. 1961; Maragos, C.M. et al, 1991; Wink D.A., et al. 1991; Hrabie, et al. 1993; Robak, et al., 1992; Moilanen, E. et al. 1993; Corell, T. et al. 1994; Karup, G. et al. 1994; Malo-Ranta, U. et al. 1994; Ma H.T. et al. 1999). These type of compounds produce NO which can stimulate the soluble guanylyl cyclase which leads to a cGMP production. Figure 1 shows the cGMP production after stimulating the soluble guanylyl cyclase with DEA NONOate in a dose dependent manner. For detection of cGMP an AlphaScreen competition assay was used.

Example 5: Inhibition of different NOS enzymes using standard inhibitors:

Table 1 summarises the results obtained by employing the assay described above and testing standard NOS inhibitors.

| enzyme | AMT [$\mu$M] | ODQ [$\mu$M] | L-NMMA [$\mu$M] | 1400W [$\mu$M] | L-NIL [$\mu$M] |
|---|---|---|---|---|---|
| iNOS | $1.3 \times 10^{-8}$ | $7.7 \times 10^{-7}$ | $3.2 \times 10^{-6}$ | $9.7 \times 10^{-7}$ | $2.2 \times 10^{-6}$ |
| eNOS | $9.9 \times 10^{-8}$ | $5.8 \times 10^{-7}$ | $2.3 \times 10^{-6}$ | $2.0 \times 10^{-4}$ | $2.7 \times 10^{-5}$ |
| nNOS | $3.1 \times 10^{-8}$ | $9.0 \times 10^{-7}$ | $7.9 \times 10^{-6}$ | $1.5 \times 10^{-5}$ | $2.8 \times 10^{-4}$ |

In Table 1 is given: IC50 [$\mu$M] determination of different compounds for the different NOS enzymes.

Above mentioned NOS inhibitors are state of the art as depicted below:

AMT: Nakane, M. et al., 1995; Tracey, W.R. et al., 1995; Rairigh, R.L. et al.1998; Briones, A-M. et al., 1999;

ODQ: Boulton, C. L. et al., 1995; Brunner, F. et al., 1995; Garthwaite, J et al., 1995; Moro, M.A. et al., 1996; Schrammel, A. et al., 1996; Southam, E. et al., 1996; Abi-Gerges, N. et al., 1997; Olson, L.J, et al., 1997; Sobey C.G. & Faraci F.M. 1997; Hwang, T.L. et al., 1998; R. Motterlini, et al., 2000;

L-NMMA: Sakuma,I. et al., 1988; Rees, D.D. et al., 1989;

**1400W:** Garvey, E.P et al., 1997; Thomsen, L.L. et al., 1997; Laszlo F. & Whittle B.J.R, 1997; Gumpricht, E. et al., 2002;

**L-NIL:** Moore, W.M. et al., 1994.

Literature:

Meyer B. and Hemmens B. (1997) Biosynthesis and action of nitric oxide in mammalian cells Trends Biochem. Sci. 22, 477-481

Patrick Vallance and James Leiper: Blocking NO Synthesis: How, where and why ? Nature Reviews/Drug Discovery, Volume 1 December 2002, 939-950

Wendy K. Alderton, Chris E. Cooper and Richard G. Knowles: Nitric oxide synthases: structure, function and inhibition Biochem. J. (2001) 357, 593-615

Janssens SP. Shimouchi A. Quertermous T. Bloch DB. Bloch, KD. Cloning and expression of a cDNA encoding human endothelium-derived relaxing factor/nitric oxide synthase. Journal of Biological Chemistry 267(21):14519-22, 1992 Jul 25.

Marsden PA. Schappert KT. Chen HS. Flowers M. Sundell CL. Wilcox JN. Lamas S. Michel T. Molecular cloning and characterization of human endothelial nitric oxide synthase. FEBS Letters. Vol. 307(3)(pp 287-293), 1992.

Nakane M. Schmidt HHHW. Pollock JS. Forstermann U. Murad F. Cloned human brain nitric oxide synthase is highly expressed in skeletal muscle. FEBS Letters. Vol. 316(2)(pp 175-180), 1993.

Geller DA. Lowenstein CJ. Shapiro RA. Nussler AK. Di Silvio M. Wang SC. Nakayama DK. Simmons RL. Snyder SH. Billiar TR. Molecular cloning and expression of inducible nitric oxide synthase from human hepatocytes. Proceedings of the National Academy of Sciences of the United States of America. Vol. 90(8)(pp 3491-3495), 1993.

Sherman PA. Laubach VE. Reep BR. Wood ER. Purification and cDNA sequence of an inducible nitric oxide synthase from a human tumor cell line. Biochemistry. Vol. 32(43)(pp 11600-11605), 1993.

Charles IG. Palmer RMJ. Hickery MS. Bayliss MT. Chubb AP. Hall VS. Moss DW. Moncada S. Cloning, characterization, and expression of a cDNA encoding an inducible nitric oxide synthase from the human chondrocyte. Proceedings of the National Academy of Sciences of the United States of America. Vol. 90(23)(pp 11419-11423), 1993.

Maier R. Bilbe G. Rediske J. Lotz M. Inducible nitric oxide synthase from human articular chondrocytes: cDNA cloning and analysis of mRNA expression. Biochimica et Biophysica Acta - Protein Structure & Molecular Enzymology. Vol. 1208(1)(pp 145-150), 1994.

Chartrain NA. Geller DA. Koty PP. Sitrin NF. Nussler AK. Hoffman EP. Billiar TR. Hutchinson NI. Mudgett JS. Molecular cloning, structure, and chromosomal localization of the human inducible nitric oxide synthase gene. Journal of Biological Chemistry. Vol. 269(9)(pp 6765-6772), 1994.

Griffith OW. Stuehr DJ. Nitric oxide synthases: Properties and catalytic mechanism. Annual Review of Physiology. Vol. 57(pp 707-736), 1995

Mayer, B. :Biochemistry and molecular pharmacology of nitric oxide synthases, in Nitric Oxide in the Nervous System (S.R. Vincent, ed.), Academic, New York, 21-42,1995

Masters BSS. McMittan K. Sheta EA. Nishimura JS. Roman LJ. Martasek P. Neuronal nitric oxide synthase, a modular enzyme formed by convergent evolution: Structure studies of a cysteine thiolate-liganded heme protein that hydroxylates L-arginine to produce NO. as a cellular signal. FASEB Journal. Vol. 10(5)(pp 552-558), 1996.

Marletta MA. Yoon PS. Iyengar R. Leaf CD. Wishnok JS. Macrophage oxidation of L-arginine to nitrite and nitrate: Nitric oxide is an intermediate. Biochemistry. Vol. 27(24)(pp 8706-8711), 1988.

Kwon NS. Nathan CF. Gilker C. Griffith OW. Matthews DE. Stuehr DJ. L-Citrulline production from L-arginine by macrophage nitric oxide synthase. The ureido oxygen derives from dioxygen. Journal of Biological Chemistry. Vol. 265(23)(pp 13442-13445), 1990.

Mayer B. John M. Heinzel B. Werner ER. Wachter H. Schultz G. Bohme E. Brain nitric oxide synthase is a biopterin- and flavin-containing multi-functional oxido-reductase. FEBS Letters. Vol. 288(1-2)(pp 187-191), 1991.

Stuehr DJ. Kwon NS. Nathan CF. Griffith OW. Feldman PL. Wiseman J. N(omega)-hydroxy-L-arginine is an intermediate in the biosynthesis of nitric oxide from L-arginine. Journal of Biological Chemistry. Vol. 266(10)(pp 6259-6263), 1991.

Klatt P. Schmidt K. Uray G. Mayer B. Multiple catalytic functions of brain nitric oxide synthase. Biochemical characterization, cofactor-requirement, and the role of N(omega)-hydroxy-L- arginine as an intermediate. Journal of Biological Chemistry. Vol. 268(20)(pp 14781-14787), 1993.

Feldmann PL. Griffith OW. And Stuehr DJ.: The surprising life of nitric oxide. Chem. Eng. News 71, 26-38, 1993

Marletta MA. Nitric oxide synthase: Aspects concerning structure and catalysis. Cell. Vol. 78(6)(pp 927-930), 1994.

Pufahl RA. Marletta MA. Oxidation of N(G)-hydroxy-L-arginine by nitric oxide synthase: Evidence for the involvement of the heme in catalysis. Biochemical & Biophysical Research Communications. Vol. 193(3)(pp 963-970), 1993.

Clement B. Schultze-Mosgau M-H. Wohlers H. Cytochrome P450 dependent N-hydroxylation of a guanidine (debrisoquine), microsomal catalysed reduction and further oxidation of the N-hydroxy-guanidine metabolite to the urea derivative. Similarity with the oxidation of arginine to citrulline and nitric oxide. Biochemical Pharmacology. Vol. 46 (12)(pp 2249-2267), 1993.

Powell D., Williams D. Nitric Oxide Synthase Screening Kit for the detection of inhibitors of NOS enzyme activity Life Science News, 2002 Amersham Biosiences

Misko T.P., Schilling R.J., Salvemini D., Moore W.M., Currie M.G. A; A Fluorometric Assay for the Measurement of Nitrate in Biological Samples Analytical Biochemistry 214, 11-16 1993

Gerzer R., et al.; Soluble guanylate cyclase purified from bovine lung contains heme and copper; FEBS Lett. 132, 71 (1981)

Zabel U., et al.;Human soluble guanylate cyclase: functional expression and revised isoenzyme family; Biochem. J. 335 (Pt 1), 51 (1998)

Hoenicka, M.et al.; Purified soluble guanylyl cyclase expressed in a baculovirus/Sf9 system: stimulation by YC-1, nitric oxide, and carbon monoxide; J. Mol. Med. 77, 14 (1999)

Lee, Y.C. et al.; Human recombinant soluble guanylyl cyclase: expression, purification, and regulation; PNAS 97, 10763 (2000)

Kosarikov, D.N. et al.; Human soluble guanylate cyclase: functional expression, purification and structural characterization; Arch. Biochem. Biophys. 388, 185 (2001)
Claret E.J., et al. ; A new HTRF cGMP assay for monitoring Guanylyl cyclase activity; Poster presentation at the SBS Meeting in Orlando (2004)

Perkin Elmer: Flashplate cyclic cGMP [$^{125}$I]-assay product no: SMP002J001PK Keele, B.B. Jr. et al.; Superoxide Dismutase from Escherichia coli B; JBC Vol.245, No. 22, pp6176-6181 (1970).

Beckman J. S. et al. ; Apparent hydroxyl radical production by peroxynitrite: Implications for endothelial injury from nitric oxide and superoxide; Proc. Natl. Acad. Sci USA Vol 87, pp. 1620-1624 (1990).

Drago R.S. & Paulik F.E.; The Reaction of Nitrogen(II) Oxide with Diethylamine; JACS 82, 96 (1960)

Drago R.S. & B.R. Karstetter B.R.; The Reaction of Nitrogen(II) Oxide with Various Primary and Secondary Amines; JACS 83, 1819 (1961)

Maragos, C.M. et al.; Complexes of NO with nucleophiles as agents for the controlled biological release of nitric oxide. Vasorelaxant effects; J. Med. Chem. 34, 3242 (1991)

Wink D.A., et al.; DNA deaminating ability and genotoxicity of nitric oxide and its progenitors; Science 254, 1001 (1991)

Hrabie, et al.; New nitric oxide-releasing zwitterions derived from polyamines; JOC 58, 1472 (1993)

Robak, et al.; Pharmacol. Res. 25 S2, 355 (1992)

Moilanen, E: et al.; Inhibition by nitric oxide-donors of human polymorphonuclear leucocyte functions; Br. J. Pharmacol. 109, 852 (1993)

Corell, T. et al.; Pharmacology of mesoionic oxatriazole derivatives in blood, cardiovascular and respiratory systems; Pol. J. Pharmacol. 46, 553 (1994)

Karup, G. et al.;Mesoionic oxatriazole derivatives-a new group of NO-donors; Pol. J. Pharmacol. 46, 541 (1994)

Malo-Ranta, U. et al.; Nitric oxide donor GEA 3162 inhibits endothelial cell-mediated oxidation of low density lipoprotein; FEBS Lett. 337, 179 (1994)

Ma, H.T. et al.; Ca(2+) entry activated by S-nitrosylation. Relationship to store-operated ca(2+) entry; J. Biol. Chem. 274, 35318 (1999)

Nakane, M. et al.; Novel potent and selective inhibitors of inducible nitric oxide synthase; Mol. Pharmacol. 47, 831 (1995)

Tracey, W.R. et al.; In vivo pharmacological evaluation of two novel type II (inducible) nitric oxide synthase inhibitors; Can. J. Physiol. Pharmacol. 73, 665 (1995)

Rairigh, R.L. et al.; Role of inducible nitric oxide synthase in regulation of pulmonary vascular tone in the late gestation ovine fetus; J. Clin. Invest. 101, 15 (1998)

Briones, A-M. et al.; Role of iNOS in the vasodilator responses induced by L-arginine in the middle cerebral artery from normotensive and hypertensive rats; Br. J. Pharmacol. 126, 111 (1999)

Boulton, C. L. et al.; Nitric oxide-dependent long-term potentiation is blocked by a specific inhibitor of soluble guanylyl cyclase; Neuroscience 69, 699 (1995)

Brunner, F. et al.; Novel guanylyl cyclase inhibitor, ODQ reveals role of nitric oxide, but not of cyclic GMP in endothelin-1 secretion; FEBS Lett. 376, 262 (1995)

Garthwaite, J et al.; Potent and selective inhibition of nitric oxide-sensitive guanylyl cyclase by 1H-[1,2,4]oxadiazolo [4,3-a]quinoxalin-1-one; Mol. Pharmacol. 48, 184 (1995)

Moro, M.A. et al.; cGMP mediates the vascular and platelet actions of nitric oxide: confirmation using an inhibitor of the soluble guanylyl cyclase; PNAS 93, 1480 (1996)

Schrammel, A. et al.; Characterization of 1H-[1,2,4]oxadiazolo[4,3-a]quinoxalin-1-one as a heme-site inhibitor of nitric oxide-sensitive guanylyl cyclase; Mol. Pharmacol. 50, 1 (1996)

Southam, E. et al.; The nitric oxide-cyclic GMP pathway and synaptic plasticity in the rat superior cervical ganglion; Br. J. Pharmacol. 119, 527 (1996)

Abi-Gerges, N. et al. ; A comparative study of the effects of three guanylyl cyclase inhibitors on the L-type Ca2+ and muscarinic K+ currents in frog cardiac myocytes; Br. J. Pharmacol. 121, 1369 (1997)

Olson, L.J, et al.; Selective guanylyl cyclase inhibitor reverses nitric oxide-induced vasorelaxation; Hypertension 29, 254 (1997)

Sobey C.G. & Faraci F.M.; Effects of a novel inhibitor of guanylyl cyclase on dilator responses of mouse cerebral arterioles; Stroke 28, 837 (1997)

Hwang, T.L. et al.; Comparison of two soluble guanylyl cyclase inhibitors, methylene blue and ODQ, on sodium nitroprusside-induced relaxation in guinea-pig trachea; Br. J. Pharmacol 125, 1158 (1998)

Motterlini, R et al.; Endothelial heme oxygenase-1 induction by hypoxia. Modulation by inducible nitric-oxide synthase and S-nitrosothiols:; J. Biol. Chem. 275, 13613 (2000)

Sakuma,I. et al.; Identification of arginine as a precursor of endothelium-derived relaxing factor; PNAS 85, 8664 (1988)

Rees, D.D. et al.; A specific inhibitor of nitric oxide formation from L-arginine attenuates endothelium-dependent relaxation; Br. J. Pharmacol. 96, 418 (1989)

Garvey, E.P et al.; 1400W is a slow, tight binding, and highly selective inhibitor of inducible nitric-oxide synthase in vitro and in vivo; J. Biol. Chem. 272, 4959 (1997)

Thomsen, L.L. et al.; Selective inhibition of inducible nitric oxide synthase inhibits tumor growth in vivo: studies with 1400W, a novel inhibitor; Cancer Res. 57, 3300 (1997)

Laszlo F. & Whittle B.J.R.; Actions of isoform-selective and non-selective nitric oxide synthase inhibitors on endotoxin-induced vascular leakage in rat colon; Eur. J. Pharmacol. 334, 99 (1997)

Gumpricht, E. et al.; Nitric Oxide Ameliorates Hydrophobic Bile Acid-induced Apoptosis in Isolated Rat Hepatocytes by Non-mitochondrial Pathways; J. Biol. Chem. 277, 25823 (2002)

Moore, W.M. et al.; L-N6-(1-iminoethyl)lysine: a selective inhibitor of inducible nitric oxide synthase; J. Med. Chem. 37, 3886 (1994)

Description of Figure 1:

Figure 1 shows a Dose response testing of the synthetic NO donor DEA NONOate on the stimulation of soluble guanylyl cyclase and detection of the subsequent cGMP production of guanylyl cyclase by employing the AlphaScreen cGMP competition assay outlined in Example 4.

**Claims**

1.  A method for measuring nitric oxide synthase activity, which is suitable for an high throughput screening (HTS) assay type format, **characterized in that** the method comprises:

    (a) providing a solution comprising a nitric oxide synthase and a suitable substrate under conditions which allow synthesis of nitric oxide;
    (a) adding guanylyl cyclase and GTP under conditions which allow synthesis of cGMP;
    (c) measuring cGMP with an antibody capable of detecting cGMP.

2.  A method for determining whether a substance is modulator of nitric oxide synthase activity, which is suitable for an high throughput screening (HTS) assay type format, **characterized in that** the method comprises:

    (a) providing a first solution comprising a nitric oxide synthase and a suitable substrate under conditions which allow synthesis of nitric oxide;
    (b) providing a second solution comprising a nitric oxide synthase and a suitable substrate under conditions which allow synthesis of nitric oxide;
    (c) adding a substance to be tested to the first or the second solution;
    (d) adding guanylyl cyclase and GTP under conditions which allow synthesis of cGMP to the first and the second solution;

(e) measuring cGMP with an antibody capable of detecting cGMP in the first and the second solution;

(f) comparing cGMP levels of the first and the second solution, wherein a modulator of a nitric oxide synthase activity is identified by different levels of cGMP.

3. A method for detection of nitric oxide (NO), which is suitable for an high throughput screening (HTS) assay type format, **characterized in that** the method comprises:

(a) providing a solution comprising NO;

(b) adding guanylyl cyclase and guanosin triphosphate (GTP) under conditions which allow systhesis of cyclic guanosin monophosphate (cGMP);

(c) measuring cGMP with an antibody capable of detecting cGMP.

4. A method according to any one of claims 1-3 in which the substrate is arginine.

5. A method according to any one of claims 1-4 in which the nitric oxide synthase is selected from a group consisting of: i-NOS, e-NOS, and n-NOS.

6. A method according to claim 2 in which the modulator is an inhibitor of the nitric oxide synthase activity, wherein an inhibitor is identified by an reduced level of cGMP which can be measured in the solution to which the substance in step (c) was added.

7. A method according to claim 2 in which the modulator is an activator of the nitric oxide synthase activity, wherein an activator is identified by an enhanced level of cGMP which can be measured in the solution to which the substance in step (c) was added.

8. A method according to claim 1, 2, 3, 4, 5, 6 or 7 which is in an high throughput (HTS) format.

9. A method according to any one of claim 1, 2, 3, 6, 7 or 8 **characterised in that** superoxide dismutase is added together with guanylyl cyclase and GTP.

10. A method according to claim 8 in which a plate having at least 96 wells is used.

11. A method according to claim 10 in which a 384 well plate or a 1536 well plate is used.

12. A method according to claim 8 in which a chip is used as reaction and/or readout platform.

13. Use of a method according to any one of claim 1, 2, 3, 4, 5, 6, 7, 8, 9, 10, 11 or 12 in a high throughput screening.

14. A kit for determining whether a substance is a modulator of NOS activity comprising

(a) guanylyl cyclase,

(b) GTP, and

(c) an antibody which is capable of binding to cGMP.

15. A kit for measuring nitric oxide synthase (NOS) activity comprising:

(a) guanylyl cyclase,

(b) GTP, and

(c) an antibody which is capable of binding to cGMP.

16. A kit for detection of nitric oxide (NO) comprising:

(a) guanylyl cyclase,

(b) GTP, and

(c) an antibody which is capable of binding to cGMP.

17. A kit according to claim 14 or 15 which additionally comprises a nitric oxide synthase (NOS) which is selected from a group consisting of: I-NOS, e-NOS, and n-NOS.

**18.** A kit according to claim 14, 15, 16 or 17 comprising additionally superoxid dismutase.

**Patentansprüche**

**1.** Verfahren zur Messung der Stickoxid-synthase-Aktivität, das sich für ein Screening-Testformat mit hohem Durchsatz (HTS) eignet, **dadurch gekennzeichnet, dass** das Verfahren umfasst:

   (a) das Bereitstellen einer Lösung, die Stickoxid-synthase und ein geeignetes Substrat umfasst, unter Bedingungen, die eine Synthese von Stickoxid ermöglichen;
   (b) das Zugeben von Guanylyl-cyclase und GTP unter Bedingungen, die eine Synthese von cGMP ermöglichen;
   (c) das Messen von cGMP mit einem Antikörper, der zum Nachweis von cGMP befähigt ist.

**2.** Verfahren zur Feststellung, ob eine Substanz einen Modulator von Stickoxid-synthase-Aktivität darstellt, das sich für ein Screening-Testformat mit hohem Durchsatz (HTS) eignet, **dadurch gekennzeichnet, dass** das Verfahren umfasst:

   (a) das Bereitstellen einer ersten Lösung, die Stickoxid-synthase und ein geeignetes Substrat umfasst, unter Bedingungen, die eine Synthese von Stickoxid ermöglichen;
   (b) das Bereitstellen einer zweiten Lösung, die Stickoxid-synthase und ein geeignetes Substrat umfasst, unter Bedingungen, die eine Synthese von Stickoxid ermöglichen;
   (c) das Zugeben einer zu testenden Substanz zur ersten oder zur zweiten Lösung;
   (d) das Zugeben von Guanylyl-cyclase und GTP unter Bedingungen, die eine Synthese von cGMP ermöglichen, zur ersten und zur zweiten Lösung;
   (e) das Messen von cGMP mit einem Antikörper, der zum Nachweis von cGMP befähigt ist, in der ersten und der zweiten Lösung;
   (f) das Vergleichen der cGMP-Konzentrationen der ersten und der zweiten Lösung, wobei ein Modulator der Stickoxid-synthase-Aktivität durch unterschiedliche Konzentrationen an cGMP identifiziert wird.

**3.** Verfahren zum Nachweis von Stickoxid (NO), das sich für ein Screening-Testformat mit hohem Durchsatz (HTS) eignet, **dadurch gekennzeichnet, dass** das Verfahren umfasst:

   (a) das Bereitstellen einer Lösung, die NO umfasst;
   (b) das Zugeben von Guanylyl-cyclase und Guanosin-triphosphat (GTP) unter Bedingungen, die eine Synthese von cyclischem Guanosinmonophosphat (cGMP) ermöglichen;
   (c) das Messen von cGMP mit einem Antikörper, der zum Nachweis von cGMP befähigt ist.

**4.** Verfahren nach einem der Ansprüche 1 bis 3, wobei es sich bei dem Substrat um Arginin handelt.

**5.** Verfahren nach einem der Ansprüche 1 bis 4, wobei die Stickoxid-synthase aus einer Gruppe ausgewählt ist, die besteht aus i-NOS, e-NOS und n-NOS.

**6.** Verfahren nach Anspruch 2, wobei es sich bei dem Modulator um einen Inhibitor der Stickoxid-synthase-Aktivität handelt, wobei ein Inhibitor durch eine verringerte Konzentration an cGMP identifiziert wird, das in der Lösung, zu der in Stufe (c) die Substanz gegeben worden ist, gemessen werden kann.

**7.** Verfahren nach Anspruch 2, wobei es sich bei dem Modulator um einen Aktivator der Stickoxid-synthase-Aktivität handelt, wobei ein Aktivator durch eine erhöhte Konzentration an cGMP identifiziert wird, das in der Lösung, zu der in Stufe (c) die Substanz gegeben worden ist, gemessen werden kann.

**8.** Verfahren nach Anspruch 1, 2, 3, 4, 5, 6 oder 7, das in einem Format mit hohem Durchsatz (HTS) durchgeführt wird.

**9.** Verfahren nach einem der Ansprüche 1, 2, 3, 6, 7 oder 8, **dadurch gekennzeichnet, dass** Superoxid-dismutase zusammen mit Guanylyl-cyclase und GTP zugegeben wird.

**10.** Verfahren nach Anspruch 8, in dem eine Platte mit mindestens 96 Vertiefungen verwendet wird.

**11.** Verfahren nach Anspruch 10, in dem eine Platte mit 384 Vertiefungen oder eine Platte mit 1536 Vertiefungen verwendet wird.

**12.** Verfahren nach Anspruch 8, in dem ein Chip als Plattform für die Reaktion und/oder das Auslesen verwendet wird.

**13.** Verwendung nach einem der Ansprüche 1, 2, 3, 4, 5, 6, 7, 8, 9, 10, 11 oder 12 bei einem Screening mit hohem Durchsatz.

**14.** Kit zur Bestimmung, ob eine Substanz einen Modulator der NOS-Aktivität darstellt, umfassend:

(a) Guanylyl-cyclase,
(b) GTP und
(c) einen Antikörper, der zur Bindung an cGMP befähigt ist.

**15.** Kit zur Messung der Stickoxid-synthase (NOS)-Aktivität, umfassend:

(a) Guanylyl-cyclase,
(b) GTP und
(c) einen Antikörper, der zur Bindung an cGMP befähigt ist.

**16.** Kit zum Nachweis von Stickoxid (NO), umfassend:

(a) Guanylyl-cyclase,
(b) GTP und
(c) einen Antikörper, der zur Bindung an cGMP befähigt ist.

**17.** Kit nach Anspruch 14 oder 15, der zusätzlich eine Stickoxid-synthase (NOS) umfasst, die aus der Gruppe bestehend aus: i-NOS, e-NOS und n-NOS ausgewählt ist.

**18.** Kit nach Anspruch 14, 15, 16 oder 17, zusätzlich umfassend Superoxid-dismutase.


**Revendications**

**1.** Procédé de mesure de l'activité d'une oxyde nitrique synthase, qui est adapté au format d'un essai de type criblage à haut débit (HTS), **caractérisé en ce que** le procédé comprend :

(a) la fourniture d'une solution comprenant une oxyde nitrique synthase et un substrat approprié dans des conditions permettant la synthèse d'oxyde nitrique ;
(b) l'ajout de guanylyle cyclase et de GTP dans des conditions qui permettent la synthèse de GMPc ;
(c) la mesure du GMPc avec un anticorps capable de détecter le GMPc.

**2.** Procédé permettant de déterminer si une substance est un modulateur de l'activité d'une oxyde nitrique synthase, qui est adapté au format d'un essai de type criblage à haut débit (HTS), **caractérisé en ce que** le procédé comprend :

(a) la fourniture d'une première solution comprenant une oxyde nitrique synthase et un substrat approprié dans des conditions permettant la synthèse d'oxyde nitrique ;
(b) la fourniture d'une seconde solution comprenant une oxyde nitrique synthase et un substrat approprié dans des conditions permettant la synthèse d'oxyde nitrique ;
(c) l'ajout d'une substance à tester à la première solution ou à la seconde solution ;
(d) l'ajout de guanylyle cyclase et de GTP dans des conditions qui permettent la synthèse de GMPc à la première solution et à la seconde solution ;
(e) la mesure du GMPc avec un anticorps capable de détecter le GMPc dans la première solution et la seconde solution ;
(f) la comparaison des taux de GMPc de la première solution et de la seconde solution, un modulateur de l'activité d'une oxyde nitrique synthase étant identifié par différents taux de GMPc.

**3.** Procédé de détection d'oxyde nitrique (NO), qui est adapté au format d'un essai de type criblage à haut débit (HTS), **caractérisé en ce que** le procédé comprend :

(a) la fourniture d'une solution comprenant du NO ;
(b) l'ajout de guanylyle cyclase et de guanosine triphosphate (GTP) dans des conditions qui permettent la synthèse de guanosine monophosphate cyclique (GMPc) ;
(c) la mesure du GMPc avec un anticorps capable de détecter le GMPc.

**4.** Procédé selon l'une quelconque des revendications 1 à 3, dans lequel le substrat est l'arginine.

**5.** Procédé selon l'une quelconque des revendications 1 à 4, dans lequel l'oxyde nitrique synthase est choisie dans le groupe constitué par : i-NOS, e-NOS et n-NOS.

**6.** Procédé selon la revendication 2, dans lequel le modulateur est un inhibiteur de l'activité de l'oxyde nitrique synthase, un inhibiteur étant identifié par un taux réduit de GMPc pouvant être mesuré dans la solution à laquelle la substance de l'étape (c) a été ajoutée.

**7.** Procédé selon la revendication 2, dans lequel le modulateur est un activateur de l'activité de l'oxyde nitrique synthase, un activateur étant identifié par un taux accru de GMPc pouvant être mesuré dans la solution à laquelle la substance de l'étape (c) a été ajoutée.

**8.** Procédé selon la revendication 1, 2, 3, 4, 5, 6 ou 7, lequel est sous un format à haut débit (HTS).

**9.** Procédé selon l'une quelconque des revendications 1, 2, 3, 6, 7 ou 8, **caractérisé en ce qu'**une superoxyde dismutase est ajoutée avec la guanylyle cyclase et le GTP.

**10.** Procédé selon la revendication 8, dans lequel une plaque possédant au moins 96 puits est utilisée.

**11.** Procédé selon la revendication 10, dans lequel une plaque à 384 puits ou une plaque à 1536 puits est utilisée.

**12.** Procédé selon la revendication 8, dans lequel une puce est utilisée comme plate-forme de réaction et/ou de lecture.

**13.** Utilisation d'un procédé selon l'une quelconque des revendications 1, 2, 3, 4, 5, 6, 7, 8, 9, 10, 11 ou 12 dans un criblage à haut débit.

**14.** Kit pour déterminer si une substance est un modulateur de l'activité NOS, comprenant :

(a) guanylyle cyclase,
(b) GTP, et
(c) un anticorps capable de se lier au GMPc.

**15.** Kit pour mesurer l'activité d'une oxyde nitrique synthase (NOS), comprenant

(a) guanylyle cyclase,
(b) GTP, et
(c) un anticorps capable de se lier au GMPc.

**16.** Kit pour détecter de l'oxyde nitrique (NO), comprenant

(a) guanylyle cyclase,
(b) GTP, et
(c) un anticorps capable de se lier au GMPc.

**17.** Kit selon la revendication 14 ou 15, comprenant en outre une oxyde nitrique synthase (NOS) qui est choisie dans un groupe constitué par : i-NOS, e-NOS et n-NOS.

**18.** Kit selon la revendication 14, 15, 16 ou 17, comprenant en outre une superoxyde dismutase.

Figure 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MAYER et al.** *J. Biol. chem.,* 1998, vol. 273 (6), 326A-327O
- **MEYER B. ; HEMMENS B.** Biosynthesis and action of nitric oxide in mammalian cells. *Trends Biochem. Sci.,* 1997, vol. 22, 477-481
- **PATRICK VALLANCE ; JAMES LEIPER.** Blocking NO Synthesis: How, where and why ?. *Nature Reviews/Drug Discovery,* December 2002, vol. 1, 939-950
- **WENDY K. ALDERTON ; CHRIS E. COOPER ; RICHARD G. KNOWLES.** Nitric oxide synthases: structure, function and inhibition. *Biochem. J.,* 2001, vol. 357, 593-615
- **JANSSENS SP. ; SHIMOUCHI A. ; QUERTERMOUS T. ; BLOCH DB. ; BLOCH, KD.** Cloning and expression of a cDNA encoding human endothelium-derived relaxing factor/nitric oxide synthase. *Journal of Biological Chemistry,* 25 July 1992, vol. 267 (21), 14519-22
- **MARSDEN PA. ; SCHAPPERT KT. ; CHEN HS. ; FLOWERS M. ; SUNDELL CL. ; WILCOX JN. ; LAMAS S. ; MICHEL T.** Molecular cloning and characterization of human endothelial nitric oxide synthase. *FEBS Letters,* 1992, vol. 307 (3), 287-293
- **NAKANE M. ; SCHMIDT HHHW. ; POLLOCK JS. ; FORSTERMANN U. ; MURAD F.** Cloned human brain nitric oxide synthase is highly expressed in skeletal muscle. *FEBS Letters,* 1993, vol. 316 (2), 175-180
- **GELLER DA. ; LOWENSTEIN CJ. ; SHAPIRO RA. ; NUSSLER AK. ; DI SILVIO M. ; WANG SC ; NAKAYAMA DK. ; SIMMONS RL. ; SNYDER SH. ; BILLIAR TR.** Molecular cloning and expression of inducible nitric oxide synthase from human hepatocytes. *Proceedings of the National Academy of Sciences of the United States of America,* 1993, vol. 90 (8), 3491-3495
- **SHERMAN PA. ; LAUBACH VE. ; REEP BR. ; WOOD ER.** Purification and cDNA sequence of an inducible nitric oxide synthase from a human tumor cell line. *Biochemistry,* 1993, vol. 32 (43), 11600-11605
- **CHARLES IG. ; PALMER RMJ. ; HICKERY MS. ; BAYLISS MT. ; CHUBB AP. ; HALL VS. ; MOSS DW. ; MONCADA S.** Cloning, characterization, and expression of a cDNA encoding an inducible nitric oxide synthase from the human chondrocyte. *Proceedings of the National Academy of Sciences of the United States of America,* 1993, vol. 90 (23), 11419-11423
- **MAIER R. ; BILBE G. ; REDISKE J. ; LOTZ M.** Inducible nitric oxide synthase from human articular chondrocytes: cDNA cloning and analysis of mRNA expression. *Biochimica et Biophysica Acta - Protein Structure & Molecular Enzymology,* 1994, vol. 1208 (1), 145-150
- **CHARTRAIN NA. ; GELLER DA. ; KOTY PP. ; SITRIN NF. ; NUSSLER AK. ; HOFFMAN EP. ; BILLIAR TR. ; HUTCHINSON NI. ; MUDGETT JS.** Molecular cloning, structure, and chromosomal localization of the human inducible nitric oxide synthase gene. *Journal of Biological Chemistry,* 1994, vol. 269 (9), 6765-6772
- **GRIFFITH OW. ; STUEHR DJ.** Nitric oxide synthases: Properties and catalytic mechanism. *Annual Review of Physiology,* 1995, vol. 57, 707-736
- Biochemistry and molecular pharmacology of nitric oxide synthases. **MAYER, B.** Nitric Oxide in the Nervous System. Academic, 1995, 21-42
- **MASTERS BSS. ; MCMITTAN K. ; SHETA EA. ; NISHIMURA JS. ; ROMAN LJ. ; MARTASEK P.** Neuronal nitric oxide synthase, a modular enzyme formed by convergent evolution: Structure studies of a cysteine thiolate-liganded heme protein that hydroxylates L-arginine to produce NO. as a cellular signal. *FASEB Journal.,* 1996, vol. 10 (5), 552-558
- **MARLETTA MA. ; YOON PS. ; IYENGAR R. ; LEAF CD. ; WISHNOK JS.** Macrophage oxidation of L-arginine to nitrite and nitrate: Nitric oxide is an intermediate. *Biochemistry,* 1988, vol. 27 (24), 8706-8711
- **KWON NS. ; NATHAN CF. ; GILKER C. ; GRIFFITH OW. ; MATTHEWS DE. ; STUEHR DJ.** L-Citrulline production from L-arginine by macrophage nitric oxide synthase. The ureido oxygen derives from dioxygen. *Journal of Biological Chemistry,* 1990, vol. 265 (23), 13442-13445

- **MAYER B. ; JOHN M. ; HEINZEL B. ; WERNER ER. ; WACHTER H. ; SCHULTZ G. ; BOHME E.** Brain nitric oxide synthase is a biopterin- and flavin-containing multi-functional oxido-reductase. *FEBS Letters,* 1991, vol. 288 (1-2), 187-191
- **STUEHR DJ. ; KWON NS. ; NATHAN CF. ; GRIFFITH OW. ; FELDMAN PL. ; WISEMAN J.** N(omega)-hydroxy-L-arginine is an intermediate in the biosynthesis of nitric oxide from L-arginine. *Journal of Biological Chemistry,* 1991, vol. 266 (10), 6259-6263
- **KLATT P. ; SCHMIDT K. ; URAY G. ; MAYER B.** Multiple catalytic functions of brain nitric oxide synthase. Biochemical characterization, cofactor-requirement, and the role of N(omega)-hydroxy-L- arginine as an intermediate. *Journal of Biological Chemistry,* 1993, vol. 268 (20), 14781-14787
- **FELDMANN PL. ; GRIFFITH OW. ; STUEHR DJ.** The surprising life of nitric oxide. *Chem. Eng. News,* 1993, vol. 71, 26-38
- **MARLETTA MA.** Nitric oxide synthase: Aspects concerning structure and catalysis. *Cell,* 1994, vol. 78 (6), 927-930
- **PUFAHL RA. ; MARLETTA MA.** Oxidation of N(G)-hydroxy-L-arginine by nitric oxide synthase: Evidence for the involvement of the heme in catalysis. *Biochemical & Biophysical Research Communications,* 1993, vol. 193 (3), 963-970
- **CLEMENT B. ; SCHULTZE-MOSGAU M-H. ; WOHLERS H.** Cytochrome P450 dependent N-hydroxylation of a guanidine (debrisoquine), microsomal catalysed reduction and further oxidation of the N-hydroxy-guanidine metabolite to the urea derivative. Similarity with the oxidation of arginine to citrulline and nitric oxide. *Biochemical Pharmacology,* 1993, vol. 46 (12), 2249-2267
- **POWELL D. ; WILLIAMS D.** Nitric Oxide Synthase Screening Kit for the detection of inhibitors of NOS enzyme activity Life Science News. *Amersham Biosiences,* 2002
- **MISKO T.P. ; SCHILLING R.J. ; SALVEMINI D. ; MOORE W.M. ; CURRIE M.G. A.** A Fluorometric Assay for the Measurement of Nitrate. *Biological Samples Analytical Biochemistry,* 1993, vol. 214, 11-16
- **GERZER R. et al.** Soluble guanylate cyclase purified from bovine lung contains heme and copper. *FEBS Lett.,* 1981, vol. 132, 71
- **ZABEL U. et al.** Human soluble guanylate cyclase: functional expression and revised isoenzyme family. *Biochem. J.,* 1998, vol. 335 (1), 51
- **HOENICKA, M. et al.** Purified soluble guanylyl cyclase expressed in a baculovirus/Sf9 system: stimulation by YC-1, nitric oxide, and carbon monoxide. *J. Mol. Med.,* 1999, vol. 77, 14
- **LEE, Y.C. et al.** Human recombinant soluble guanylyl cyclase: expression, purification, and regulation. *PNAS,* 2000, vol. 97, 10763
- **KOSARIKOV, D.N. et al.** Human soluble guanylate cyclase: functional expression, purification and structural characterization. *Arch. Biochem. Biophys.,* 2001, vol. 388, 185
- **CLARET E.J. et al.** *A new HTRF cGMP assay for monitoring Guanylyl cyclase activity,* 2004
- **KEELE, B.B. JR. et al.** Superoxide Dismutase from Escherichia coli B. *JBC,* 1970, vol. 245 (22), 6176-6181
- **BECKMAN J. S. et al.** Apparent hydroxyl radical production by peroxynitrite: Implications for endothelial injury from nitric oxide and superoxide. *Proc. Natl. Acad. Sci USA,* 1990, vol. 87, 1620-1624
- **DRAGO R.S. ; PAULIK F.E.** The Reaction of Nitrogen(II) Oxide with Diethylamine. *JACS,* 1960, vol. 82, 96
- **DRAGO R.S. ; B.R. KARSTETTER B.R.** The Reaction of Nitrogen(II) Oxide with Various Primary and Secondary Amines. *JACS,* 1961, vol. 83, 1819
- **MARAGOS, C.M. et al.** Complexes of NO with nucleophiles as agents for the controlled biological release of nitric oxide. Vasorelaxant effects. *J. Med. Chem.,* 1991, vol. 34, 3242
- **WINK D.A. et al.** DNA deaminating ability and genotoxicity of nitric oxide and its progenitors. *Science,* 1991, vol. 254, 1001
- **HRABIE et al.** New nitric oxide-releasing zwitterions derived from polyamines. *JOC,* 1993, vol. 58, 1472
- **ROBAK et al.** *Pharmacol. Res.,* 1992, vol. 25 S2, 355
- **MOILANEN, E et al.** Inhibition by nitric oxide-donors of human polymorphonuclear leucocyte functions. *Br. J. Pharmacol.,* 1993, vol. 109, 852
- **CORELL, T. et al.** Pharmacology of mesoionic oxatriazole derivatives in blood, cardiovascular and respiratory systems. *Pol. J. Pharmacol.,* 1994, vol. 46, 553
- **KARUP, G. et al.** Mesoionic oxatriazole derivatives-a new group of NO-donors. *Pol. J. Pharmacol.,* 1994, vol. 46, 541
- **MALO-RANTA, U. et al.** Nitric oxide donor GEA 3162 inhibits endothelial cell-mediated oxidation of low density lipoprotein. *FEBS Lett.,* 1994, vol. 337, 179
- **MA, H.T. et al.** Ca(2+) entry activated by S-nitrosylation. Relationship to store-operated ca(2+) entry. *J. Biol. Chem.,* 1999, vol. 274, 35318
- **NAKANE, M. et al.** Novel potent and selective inhibitors of inducible nitric oxide synthase. *Mol. Pharmacol.,* 1995, vol. 47, 831
- **TRACEY, W.R. et al.** In vivo pharmacological evaluation of two novel type II (inducible) nitric oxide synthase inhibitors. *Can. J. Physiol. Pharmacol.,* 1995, vol. 73, 665

- **RAIRIGH, R.L. et al.** Role of inducible nitric oxide synthase in regulation of pulmonary vascular tone in the late gestation ovine fetus. *J. Clin. Invest.,* 1998, vol. 101, 15
- **BRIONES, A-M. et al.** Role of iNOS in the vasodilator responses induced by L-arginine in the middle cerebral artery from normotensive and hypertensive rats. *Br. J. Pharmacol.,* 1999, vol. 126, 111
- **BOULTON, C. L. et al.** Nitric oxide-dependent long-term potentiation is blocked by a specific inhibitor of soluble guanylyl cyclase. *Neuroscience,* 1995, vol. 69, 699
- **BRUNNER, F. et al.** Novel guanylyl cyclase inhibitor, ODQ reveals role of nitric oxide, but not of cyclic GMP in endothelin-1 secretion. *FEBS Lett.,* 1995, vol. 376, 262
- **GARTHWAITE, J et al.** Potent and selective inhibition of nitric oxide-sensitive guanylyl cyclase by 1H-[1,2,4]oxadiazolo[4,3-a]quinoxalin-1-one. *Mol. Pharmacol.,* 1995, vol. 48, 184
- **MORO, M.A. et al.** cGMP mediates the vascular and platelet actions of nitric oxide: confirmation using an inhibitor of the soluble guanylyl cyclase. *PNAS,* 1996, vol. 93, 1480
- **SCHRAMMEL, A. et al.** Characterization of 1H-[1,2,4]oxadiazolo[4,3-a]quinoxalin-1-one as a heme-site inhibitor of nitric oxide-sensitive guanylyl cyclase. *Mol. Pharmacol.,* 1996, vol. 50, 1
- **SOUTHAM, E. et al.** The nitric oxide-cyclic GMP pathway and synaptic plasticity in the rat superior cervical ganglion. *Br. J. Pharmacol.,* 1996, vol. 119, 527
- **ABI-GERGES, N. et al.** A comparative study of the effects of three guanylyl cyclase inhibitors on the L-type Ca2+ and muscarinic K+ currents in frog cardiac myocytes. *Br. J. Pharmacol.,* 1997, vol. 121, 1369
- **OLSON, L.J et al.** Selective guanylyl cyclase inhibitor reverses nitric oxide-induced vasorelaxation. *Hypertension,* 1997, vol. 29, 254
- **SOBEY C.G. ; FARACI F.M.** Effects of a novel inhibitor of guanylyl cyclase on dilator responses of mouse cerebral arterioles. *Stroke,* 1997, vol. 28, 837
- **HWANG, T.L. et al.** Comparison of two soluble guanylyl cyclase inhibitors, methylene blue and ODQ, on sodium nitroprusside-induced relaxation in guinea-pig trachea. *Br. J. Pharmacol,* 1998, vol. 125, 1158
- **MOTTERLINI, R et al.** Endothelial heme oxygenase-1 induction by hypoxia. Modulation by inducible nitric-oxide synthase and S-nitrosothiols. *J. Biol. Chem.,* 2000, vol. 275, 13613
- **SAKUMA,I. et al.** Identification of arginine as a precursor of endothelium-derived relaxing factor. *PNAS,* 1988, vol. 85, 8664
- **REES, D.D. et al.** A specific inhibitor of nitric oxide formation from L-arginine attenuates endothelium-dependent relaxation. *Br. J. Pharmacol.,* 1989, vol. 96, 418
- **GARVEY, E.P et al.** 1400W is a slow, tight binding, and highly selective inhibitor of inducible nitric-oxide synthase in vitro and in vivo. *J. Biol. Chem.,* 1997, vol. 272, 4959
- **THOMSEN, L.L. et al.** Selective inhibition of inducible nitric oxide synthase inhibits tumor growth in vivo: studies with 1400W, a novel inhibitor. *Cancer Res.,* 1997, vol. 57, 3300
- **LASZLO F. ; WHITTLE B.J.R.** Actions of isoform-selective and non-selective nitric oxide synthase inhibitors on endotoxin-induced vascular leakage in rat colon. *Eur. J. Pharmacol.,* 1997, vol. 334, 99
- **GUMPRICHT, E. et al.** Nitric Oxide Ameliorates Hydrophobic Bile Acid-induced Apoptosis in Isolated Rat Hepatocytes by Non-mitochondrial Pathways. *J. Biol. Chem.,* 2002, vol. 277, 25823
- **MOORE, W.M. et al.** L-N6-(1-iminoethyl)lysine: a selective inhibitor of inducible nitric oxide synthase. *J. Med. Chem.,* 1994, vol. 37, 3886